# EUROPEAN PATENT APPLICATION

(11) **EP 2 594 649 A1**
(43) Date of publication of application: **22.05.2013**
(21) Application number: 12188273.2
(22) Date of filing: 12.10.2012
(51) Int. Cl.: C12Q 1/68

(54) **A method and a kit to predict response to antidepressant treatment**

(30) Priority: 13.10.2011 US 201161546607 P
(71) Applicant: University of Tartu, 50090 Tartu (EE)
(72) Inventor: Tammiste, Anu, 51007 Tartu (EE); Milani, Lili, 50409 Tartu (EE); Fischer, Krista, 50304 Tartu (EE); Esko, Tõnu, 50107 Tartu (EE); Pettai, Kristi, 50704 Tartu (EE); Krjutskov, Kaarel, 51009 Tartu (EE); Mägi, Reedik, 50415 Tartu (EE); Leego, Merike, 50304 Tartu (EE); Maron, Eduard, 10111 Tallinn (EE); Metspalu, Andres, 50103 Tartu (EE)
(74) Representative: Kahu, Sirje

(57) **Abstract**

The present invention relates to the method for predicting the pharmacoresponse of antidepressants in patients diagnosed with major depression and panic disorder. According to the invention, SNP (rs41271330) in the coding region of the BMP5 gene together with the markers in linkage disequilibrium with this SNP could be used as predictors of SSRI treatment efficacy. The knowledge obtained by using the method disclosed in present invention has potential to increase the efficacy of pharmacological treatment in patients with major depression and panic disorder. The invention also provides a kit for detecting individual who are likely to respond to antidepressant treatment.

SEQUENCE LISTING

<110> University of Tartu

<120> A method and a kit to predict response to antidepressant
      treatment

<130> 1835TTU 01 UTL

<150> 61546607
<151> 2011-10-13

<160> 10

<170> PatentIn version 3.5

<210> 1
<211> 1001
<212> DNA
<213> artificial sequence

<220>
<223> chemically synthetised


<220>
<221> misc_feature
<222> (1)..(1001)
<223> sequence 500bp upstream and 500 bp downsteream of rs 41271330

<220>
<221> misc_feature
<222> (501)..(501)
<223> rs 41271330 n is G or A

<400> 1
tgaggaagtt agctcagcaa gtcatgtagc tcagttctgg ctagattgtg ttgattagcc 60

atttcataat tttattactg tcatcttgac tttaatttga actgaatctt tcaacatatg 120

aaggcaactc atatgtattt taaaatgaat tactgagtga ttcactgatg aaacattctg 180

ggatgctaga aacatcactg gaagatttaa tgtaccatcc gaaggtatac atagagggta 240

aatttattgt ggctacttga tttgcagcat acgaccccat aactctctgt gcataactgc 300

tgctcgggtt tattcttacc tccaacactg gacattctgg aggagtcctg atgagagctg 360

gatttattgc ggttttgatt ttttcgtttg ttggctgctc tcacggatcg aagaagtacc 420

tcactcgcct tgaagaaggc caccatgaat ggttgttttg actgaggtcc ctgtcttccc 480

acaagaccag cagattttac nttgatactg cgtcctagaa cgtaatacaa aagcacttgg 540

tttatgaaaa agaaagaaca attacctgat gtgaatgaat tctgagggta ctttgaaaag 600

gggaaaagtt ttaaaacatt atcagaatgc cttccttaca ggccaagatt cttataaata 660

ttgaggctta ctattacaga caattatcca tctatggtgt tacatcttgc atcattcttc 720

caccacagaa taccctccag ctgctttcac tcactgagag cttctcagac cttgaagtca 780

agaatactat aaattggaag atttgactac tcattcgcct ggaaatgtcc acattaaata 840

acaatttaaa aaaatattag ctatgggctc ttcggcacta aatgccctgt aattttcctt 900

taagttataa aagccgaata cttggcaggg tgtttctact ttctccagcc tattcgaagc 960

ctttcaagtg ttaattagtg gtgatagcct ttccaccttc a 1001

<210> 2
<211> 1001
<212> DNA
<213> artificial sequence

<220>
<223> chemically synthetised

<220>
<221> misc_feature
<222> (1)..(1001)
<223> complimentary sequence of SEQ ID NO:1

<220>
<221> misc_feature
<222> (501)..(501)
<223> n is C or T

<400> 2
tgaaggtgga aaggctatca ccactaatta acacttgaaa ggcttcgaat aggctggaga 60

aagtagaaac accctgccaa gtattcggct tttataactt aaaggaaaat tacagggcat 120

ttagtgccga agagcccata gctaatattt ttttaaattg ttatttaatg tggacatttc 180

caggcgaatg agtagtcaaa tcttccaatt tatagtattc ttgacttcaa ggtctgagaa 240

gctctcagtg agtgaaagca gctggagggt attctgtggt ggaagaatga tgcaagatgt 300

aacaccatag atggataatt gtctgtaata gtaagcctca atatttataa gaatcttggc 360

ctgtaaggaa ggcattctga taatgtttta aaacttttcc ccttttcaaa gtaccctcag 420

aattcattca catcaggtaa ttgttctttc tttttcataa accaagtgct tttgtattac 480

gttctaggac gcagtatcaa ngtaaaatct gctggtcttg tgggaagaca gggacctcag 540

tcaaaacaac cattcatggt ggccttcttc aaggcgagtg aggtacttct tcgatccgtg 600

agagcagcca acaaacgaaa aaatcaaaac cgcaataaat ccagctctca tcaggactcc 660

tccagaatgt ccagtgttgg aggtaagaat aaacccgagc agcagttatg cacagagagt 720

tatggggtcg tatgctgcaa atcaagtagc cacaataaat ttaccctcta tgtatacctt 780

cggatggtac attaaatctt ccagtgatgt ttctagcatc ccagaatgtt tcatcagtga 840

atcactcagt aattcatttt aaaatacata tgagttgcct tcatatgttg aaagattcag 900

ttcaaattaa agtcaagatg acagtaataa aattatgaaa tggctaatca acacaatcta 960

gccagaactg agctacatga cttgctgagc taacttcctc a 1001

<210>  3
<211>  30
<212>  DNA
<213>  artificial sequence

<220>
<223>  chemically synthetised


<220>
<221>  misc_feature
<222>  (1)..(30)
<223>  primer

<400>  3
acgttggatg tgtattacgt tctaggacgc                                        30


<210>  4
<211>  30
<212>  DNA
<213>  artificial sequence

<220>
<223>  chemically synthetised


<220>
<221>  misc_feature
<222>  (1)..(30)
<223>  primer

<400>  4
acgttggatg gttgttttga ctgaggtccc                                        30


<210>  5
<211>  23
<212>  DNA
<213>  artificial sequence

<220>
<223>  chemically synthetised


<220>
<221>  misc_feature
<222>  (1)..(23)
<223>  primer

<400>  5
ccacaagacc agcagatttt aca                                               23


<210>  6
<211>  23
<212>  DNA
<213>  artificial sequence

<220>
<223>  chemically synthetised

<220>
<221>  misc_feature
<222>  (1)..(23)
<223>  primer

<400>  6
ccacaagacc agcagatttt acg                                              23


<210>  7
<211>  24
<212>  DNA
<213>  artificial sequence

<220>
<223>  chemically synthetised


<220>
<221>  misc_feature
<222>  (1)..(24)
<223>  primer

<400>  7
ccccttttca aagtaccctc agaa                                             24


<210>  8
<211>  23
<212>  DNA
<213>  artificial sequence

<220>
<223>  chemically synthetised


<220>
<221>  misc_feature
<222>  (1)..(23)
<223>  primer

<400>  8
agtacctcac tcgccttgaa gaa                                              23


<210>  9
<211>  23
<212>  DNA
<213>  artificial sequence

<220>
<223>  chemically synthetised


<220>
<221>  misc_feature
<222>  (1)..(23)
<223>  primer

<400>  9
acgttctagg acgcagtatc aat                                              23

<210>  10
<211>  23
<212>  DNA
<213>  artificial sequence

<220>
<223>  chemically synthetised


<220>
<221>  misc_feature
<222>  (1)..(23)
<223>  primer

<400>  10
acgttctagg acgcagtatc aac                                              23

## Description

### PRIORITY

This application is nonprovisional application of US provisional application number 61/546,607 filed on October 13 2011, and which is incorporated herein in its entirety by reference.

### SEQUENCE LISTING

This application contains sequence data which is provided on a computer readable diskette and as a paper versin. The paper version of the sequence data is identical to the datea provided on the diskette.

### FIELD OF INVENTION

This invention relates to genetic markers. More specifically the invention relates to a genetic marker predicting response to antidepressant treatment. The invention relates to a method to predict response to antidepressant treatment.

### BACKGROUND OF THE INVENTION

Major depression (MD), also indicated as major depressive disorder (MDD) is one of the most prevalent and disabling diseases with poor long-term outcomes (19). According to a report from European Union, depression and its associated problems affect 2-10% of total population. Approximately two thirds of patients diagnosed with depression are women (25); another risk factor for depression is the diagnosis of some chronic disease. Major depression has been recognized as one of the leading causes of disability by WHO, so in addition to the problems caused for an individual this disorder has a huge cost for the society. At its worst, depression is associated with increased risk of suicide: approximately 15% of individuals with severe depression commit suicide and over 50% have attempted suicide (9).

Panic disorder (PD) is a common and serious psychiatric condition characterized by unexpected panic attacks and fear of their recurrence. According to epidemiological surveys the life-time prevalence of panic disorder ranges from 2 to 4%. The prevalence of PD is slightly more than twice as high in females than in males (4), and the age of onset of is usually in the mid-twenties (26). Patients with PD have an increased risk of other psychiatric as well as medical morbidity, with major depression being the most frequent concurrent diagnosis observed in 30-60% of patients with PD (12). PD is often a seriously disabling disorder causing impairment in social, personal and occupational functioning and significant loss of quality of life (3). The rate of those PD patients, who are dependent on welfare or disability benefits, exceeds 25% in some studies (10).

Selective serotonin reuptake inhibitors (SSRIs) are a class of compounds, which increase the extracellular level of neurotransmitter serotonin by inhibiting its reuptake into the presynaptic neuron. Because of their safety and efficacy the SSRIs are widely used in the treatment of major depression and anxiety disorders including panic disorder. SSRIs are the most frequently prescribed medications for the treatment of major depression. Also, SSRIs are now generally accepted as the first-line agents in the pharmacological treatment of panic disorder. The clinical popularity of the SSRIs in the treatment of panic disorder is a result of their equal or superior effectiveness when compared with standard tricyclic and benzodiazepine treatments, combined with their superior safety and side effect profile and easy-to-follow dosing regimen compared with the earlier tricyclic antidepressants. However, the efficacy of SSRI treatment in major depression and panic disorder is unsatisfactory. It is estimated that approximately one third of patients diagnosed with major depression do not achieve or maintain a response to SSRIs (22). Also, not all panic disorder patients show sufficient drug response. For example, in a three-year follow-up only 10% of patients with PD were symptom-free (18) and only 12% of PD patients were in full remission after five years (5). It is rather difficult in clinical practice to predict which patient will respond well to a specific pharmacological treatment and which will not.

Various drug groups with totally different mechanism of action are available for the treatment of depression and panic disorder. Action mechanisms include, for example, inhibition of serotonin reuptake, inhibition of noradrenaline reuptake, inhibition of dopamine reuptake, blockade of presynaptic receptors on serotoninergic neurons and inhibition of an enzyme responsible for degradation of monoamine neurotransmitters. Such a variety of drugs should potentially enable psychiatrists to choose the most beneficial drug or combination of drugs for each individual patient. Still, in everyday clinical practice there is a lack of information about which characteristics of patients could be used to predict a superior response of one particular class of drug over another.

Currently, antidepressant drugs are administered by a trial and error method. Antidepressant response takes several weeks before the potential improvement is seen and one can assess the suitability of the drug to the patient. During this time patients may experience worsening of clinical symptoms and some of them can discontinue the treatment prematurely. To minimize the suffering for the patient and costs for society it would be valuable to know whether a drug is likely to be effective and tolerable before starting the treatment.

Pharmacogenetic studies are concentrating on how an individual's genetic background influences response to drugs. The ultimate goal of such studies is to find genetic markers, which are associated with drug response. In everyday clinical practice, these markers could be then genotyped in patients before starting the treatment and that would help to decide which drug will have the most favourable response in a given patient. This would reduce the suffering caused for the patient by ineffective treatment trials and also decrease the costs for society.

To date the molecular mechanisms underlying individual treatment responses are not clearly understood, but there is increasing evidence from pharmacogenetic studies that therapeutic outcomes of antidepressants could be determined by certain genetic variations (11). Conventional association studies applying the candidate-gene strategy to identify genetic variations influencing pharmacoresponse in MDD have produced results, which are mostly inconsistent or not replicated (11). Candidate gene selection for these studies has been based mainly on the knowledge about the primary targets of antidepressants and thus the majority of the studied genes are encoding proteins involved in the serotoninergic pathway. In recent years three genome-wide association studies have been published in the field of antidepressant pharmacogenetics. These studies did not detect any markers at genome-wide significance level and the top hits were not overlapping between the studies. Also, none of these studies confirmed previously reported candidate gene associations (7,8,23). In conclusion, today there are no genetic markers available for predicting antidepressant response, which have shown similar results in different studies and could be considered for use in clinical practice.

### SUMMARY OF THE INVENTION

Therefore the present invention succeeds in conferring the following, and other not mentioned, desirable and useful benefits and objectives.

An object of this invention is to provide a method where the patient's genotype at rs41271330 is used for predicting the patient's response to antidepressant treatment.

Another object of the invention is to provide a method where the patient's genotype at rs41271330 is used for predicting the patient's response to SSRI class of drugs.

Yet another object of the invention is to provide a method where the patient's genotype at rs41271330 is used for predicting the patient's response to escitalopram.

Another object of the invention is to provide a kit for identification of individuals likely to respond to antidepressant treatment, wherein said kit comprises a genotyping assay for detecting a genetic polymorphism rs41271330 in the coding region of the BMP5 gene or a genotyping assay for detecting a polymorphism that is in linkage disequilibrium with the polymorphism rs41271330.

Still another object of the invention is to provide a kit for identification of individuals likely to respond to selective serotonin reuptake inhibitor (SSRI) treatment, wherein said kit comprises a genotyping assay for detecting a genetic polymorphism rs41271330 in the coding region of the BMP5 gene or a genotyping assay for detecting a polymorphism that is in linkage disequilibrium with the polymorphism rs41271330.

An even further object of the invention is to provide a kit for identification of individuals likely to respond to escitalopram treatment, wherein said kit comprises a genotyping assay for detecting a genetic polymorphism rs41271330 in the coding region of the BMP5 gene or a genotyping assay for detecting a polymorphism that is in linkage disequilibrium with the polymorphism rs41271330.

Another object of the invention is a method for modulating the expression of a biological molecule encoded by the BMP5 gene or by a sequence or an antisense sequence containing alleles in linkage disequilibrium with alleles at rs41271330 for changing the potential of an individual to respond to treatment with an antidepressant.

A further object of the invention is to determine potential response to antidepressant treatment comprising the steps of:
a) obtaining a biological sample from a human subject;
b) determining a presence or an absence of a major or a minor allele of polymorphism rs41271330 in coding region of each allele of the subject's BMP5 gene, or determining alleles in linkage disequilibrium with the major or the minor allele of rs41271330 at a polymorphic position in a haplotype block containing rs41271330; and
c) assessing homozygosity of the major allele of the polymorphism rs41271330 in the coding region of the BMP5 gene, wherein homozygosity of the major allele of the polymorphism rs41271330 in the coding region of the BMP5 gene, or a genotype in linkage disequilibrium with the homozygous major allele genotype, indicates potential response and lack of homozygosity of the major allele of the polymorphism rs41271330 in the coding region of the BMP5 gene indicates potential nonresponse to the antidepressant treatment.

Still another object of the invention is a method for managing therapy of a human subject comprising the steps of:
a) obtaining a biological sample from the human subject in need of antidepressant treatment;
b) determining a presence or an absence of a major or a minor allele of polymorphism rs41271330 in coding region of each allele of the subject's BMP5 gene, or determining alleles in linkage disequilibrium with the major or the minor allele of rs41271330 at a polymorphic position in the haplotype block containing rs41271330;
c) assessing homozygosity of the major allele of the polymorphism rs41271330 in the coding region of the BMP5 gene, wherein the presence of homozygous major allele of polymorphism rs41271330 in the coding region of the BMP5 gene or a genotype in linkage disequilibrium with the homozygous major allele genotype indicates potential response to selective serotonin reuptake inhibitor (SSRI) treatment ; and
d) prescribing SSRIs to the subject having homozygous major allele genotype of polymorphism rs41271330 or a genotype in linkage disequilibrium with said allele.

### SHORT DESCRIPTION OF THE FIGURES

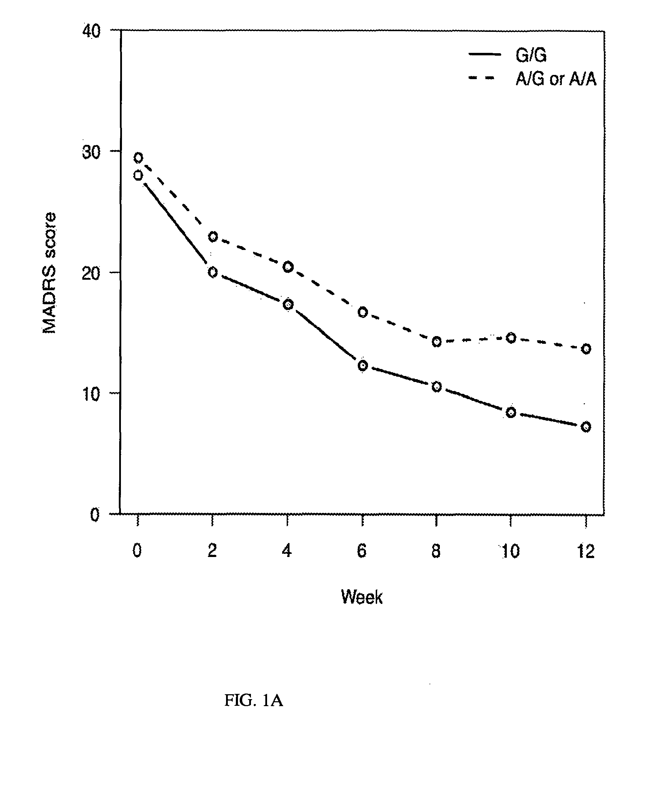

Figure 1a. Changes in the average MADRS score values during the escitalopram treatment across rs41271330 genotype groups in the Estonian MDD sample set.

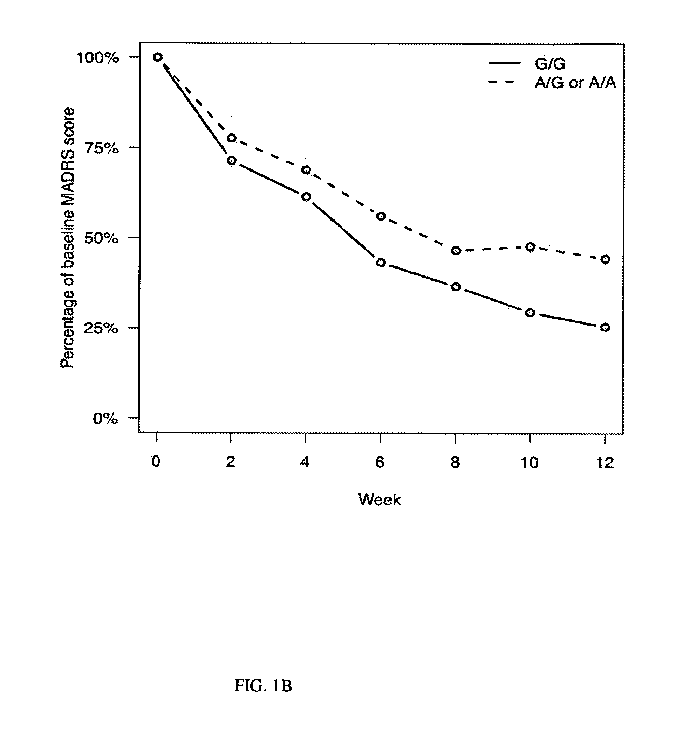

Figure 1b. Relative changes in the average MADRS score values during the escitalopram treatment across rs41271330 genotype groups in the Estonian MDD sample set.

### DESCRIPTION OF THE INVENTION

The present invention relates to the method where a genotype of the single nucleotide polymorphism rs41271330 in the coding region of BMP5 (bone morphogenetic protein 5) gene is used as a predictor of SSRI class antidepressant treatment efficacy in patients with major depression or with panic disorder. We have found that patients with major depression carrying the rs41271330 homozygous minor allele (A/A) or heterozygous minor allele (A/G) genotype are less likely to benefit from escitalopram treatment.

When considering genetic markers associated with some phenotype (for example drug response) the linkage disequilibrium (LD) between markers should be taken into account. LD refers to the phenomenon that DNA sequences, which are located close to each other in the genome, have a tendency to be inherited together. Coinherited alleles are said to be in linkage disequilibrium. Alleles in linkage disequilibrium are said to form a haplotype block. When one SNP (single nucleotide polymorphism) from a haplotype block is found to be a predictor of occurrence of some specific phenotype, all the other polymorphic positions in this haplotype block can be used as prognostic indicators of this given phenotype. Considering the present invention, it can be concluded that the SNP rs41271330 together with markers found in genome region 6p12, which are in linkage disequilibrium with this SNP, can be used as predictors of SSRI treatment efficacy in patients with major depression or panic disorder. According to Broad Institute SNAP SNP annotation and proxy search tool) there is one marker (rs57559211) that is in complete LD (r²=1.0) with rs41271330 SNP and 8 markers in strong LD (r²=0.818) with rs41271330 SNP: rs35257600, rs35695519, rs3798847, rs13216589, rs13217868, rs13219706, rs13194687, rs13204733.

In the context of present invention the term "**single-nucleotide polymorphism**" (**SNP**) means a DNA-sequence variation occurring when a single nucleotide in the genome differs between paired chromosomes in an individual.

In the context of present invention the term"**allele**" means one of two or more forms of a gene.

In the context of present invention the term **"linkage disequilibrium"** (LD) refers to the correlation between genetic variation at two or more loci or sites in the genome. Such a correlation between SNPs means that knowing the genotype of one locus might provide information about the genotype at another locus in LD with the first one. In the present invention it is assumed that genotyping of genetic variants in LD with rs41271330 will also predict antidepressant drug response and is therefore included in current invention.

In the context of present invention the term **"haplotype block"** refers to the region in the genome in which SNP pairs have correlation or genetic markers are in LD. Therefore, if one specific SNP in a haplotype block has been found to be a predicting factor of some phenotype, every other SNP in this haplotype block can be potentially used as a predictor of occurrence of this phenotype. Since population history has a large impact on patterns of LD, the boundaries of haplotype blocks can vary between different populations. The length of genomic region, which is contained in one haplotype block is usually between 5kb to 20kb.

In the context of current invention the term **"haplotype block specific to the polymorphism"** refers to the genomic region, which contains genetic markers in LD with rs41271330 and genotyping these markers in LD with rs41271330 provides also information about the genotype of the rs41271330 SNP.

In the context of current invention the term **"depressive disorders"** refers but is not limited to any kind of mood disturbances, depressive disorders, anxiety and panic disorders.

**"Modulation"** means in the current invention a process when by means of direct or indirect intervention in a cell, tissue or an organism, *in vivo, in vitro* or *ex vivo,* naturally occurring or synthetic compounds, nucleic acids, nucleic acid analogues are introduced and gene products, nucleic acids, polypeptides and/or downstream components of biological pathways are decreased or increased.

**In** the context of current invention the term **"major allele"** refers to the G allele in the case of sense strand and C allele in the case of antisense strand.

In the context of current invention the term **"minor allele"** refers to the A allele in the case of sense strand and T allele in the case of antisense strand.

**BMP5** is one of the bone morphogenetic proteins, which are members of the transforming growth factor-β family that has a role in proliferation, apoptosis and differentiation in various tissues and organs. Studies have found that Bmp5/Bmp7 double knock-out mice are embryonically lethal and double mutants were shown to have reduced size of forebrain and branchial arches, somite abnormalities and abnormal apoptosis (20). BMP5 is widely expressed in the nervous system throughout the development and in adulthood (6,17), but its biological roles are not fully understood yet. BMP5 has been shown to promote dendritic growth (2,13). In addition to that, BMP5 together with other factors has been reported to be essential for the development of major noradrenergic nucleus locus coeruleus (21,24). We have shown for the first time that the BMP5 gene could be important in the mechanism of antidepressant action and that an SNP (rs41271330) in the BMP5 gene together with the markers in linkage disequilibrium with this SNP could be used as predictors of SSRI treatment efficacy.

**SNP rs41271330** is located in the 4th exon of the BMP5 gene. It changes the guanine nucleotide into adenine in the third position of codon encoding asparagine (Asn282). This does not result in any amino acid change.

The present invention provides a method where the patient's genotype at rs41271330 is used for predicting the drug response of antidepressants.

More particularly, the present invention provides a method where the patient's genotype at rs41271330 is used for predicting the drug response of SSRI class of drugs.

In a most preferred embodiment, the present invention provides a method where the patient's genotype at rs41271330 is used for predicting the individual's drug response to escitalopram.

A person skilled in the art understands that potential treatment response to one certain substance may be very narrow, but may involve wider classes of substances, depending on the structure or mechanism of action of the provided classes of substances. Thus, response to one representative of SSRI-s may also cover other substances with similar biochemical properties, other groups of antidepressants, structurally analogous antidepressants, or even antidepressants in general.

Thus, the invention provides most generally a method for identifying an individual who is likely to respond to antidepressant treatment comprising the steps of: obtaining a biological sample from said individual; determining the individual's genotype of the BMP5 gene, wherein said determining includes assessing whether there is a nucleotide A major or minor allele present at the polymorphism rs41271330 in the coding region of each allele of said BMP5 gene, or whether there is an allele in linkage disequilibrium with major or minor allele of rs41271330 at a polymorphic position in the haplotype block containing rs41271330; evaluating whether the position of rs41271330 in the coding region of the BMP5 gene is homozygous for major allele; and determining whether said individual has potential to respond to antidepressant treatment by using information about homozygous major allele genotype of said polymorphism rs41271330 in the coding region of the BMP5 gene or a genotype in linkage disequilibrium with regards to major allele of rs41271330.

More specifically, the invention provides a method for identifying an individual who is likely to respond to the treatment with selective serotonin reuptake inhibitors, comprising the steps of: obtaining a biological sample from said individual; determining the genotype of human BMP5 gene, wherein said determining includes assessing whether there is a major or minor allele present at the polymorphism rs41271330 in the coding region of each allele of said BMP5 gene, or whether there is an allele in linkage disequilibrium with major or minor allele of rs41271330 at a polymorphic position in the haplotype block containing rs41271330; evaluating whether the position of rs41271330 in the coding region of the BMP5 gene is homozygous for major allele; and determining whether said individual is likely to respond to selective serotonin reuptake inhibitor (SSRI) treatment by using information about homozygous major allele genotype of said polymorphism rs41271330 in the coding region of the BMP5 gene or a genotype in linkage disequilibrium with regards to major allele of rs41271330.

Most preferably, the invention provides a method for identifying an individual who is likely to respond to the treatment with escitalopram, comprising the steps of: obtaining a biological sample from said individual; determining the genotype of human BMP5 gene, wherein said determining includes assessing whether there is a major or minor allele present at the polymorphism rs41271330 in the coding region of each allele of said BMP5 gene, or whether there is an allele in linkage disequilibrium with the major or the minor allele of rs41271330 at a polymorphic position in the haplotype block containing rs41271330; evaluating whether the position of rs41271330 in the coding region of the BMP5 gene is homozygous for the major allele; and determining whether said individual is likely to respond to escitalopram treatment by using information about homozygous major allele genotype at polymorphism rs41271330 in the coding region of the BMP5 gene or a genotype in linkage disequilibrium with regard to the major allele of rs41271330.

The invention also provides a method for managing the therapy of an individual comprising the steps of: obtaining a biological sample from said individual; determining the genotype of human BMP5 gene, wherein said determination includes assessing whether there is a major or minor allele present at the polymorphism rs41271330 in the coding region of each allele of said BMP5 gene, or whether there is an allele in linkage disequilibrium with a major or a minor allele of rs41271330 at a polymorphic position in the haplotype block containing rs41271330; evaluating whether the position of rs41271330 in the coding region of the BMP5 gene is homozygous for major allele; determining whether said individual is likely to respond to selective serotonin reuptake inhibitor (SSRI) treatment by using information about homozygous major allele genotype of the polymorphism rs41271330 in the coding region of the BMP5 gene or a genotype in linkage disequilibrium with regards to major allele of rs41271330; and, if said individual is likely to respond to selective serotonin reuptake inhibitor (SSRI) treatment due to the presence of homozygous major allele genotype at the polymorphism rs41271330 in the coding region of BMP5 gene or a genotype in linkage disequilibrium with said allele, prescribing to said individual a SSRI for the treatment of depressive disorders.

In a most preferred embodiment, the potential to respond to treatment to be identified is the potential to respond to escitalopram treatment.

Simultaneously, the invention provides a method for managing the therapy of an individual comprising the steps of: obtaining a biological sample from said individual; determining the genotype of human BMP5 gene, wherein said determining includes assessing whether there is a major or minor allele present at the polymorphism rs41271330 in the coding region of each allele of said BMP5 gene, or whether there is an allele in linkage disequilibrium with major or minor allele of rs41271330 at a polymorphic position in the haplotype block containing rs41271330; evaluating whether the position of rs41271330 in the coding region of the BMP5 gene is homozygous for major allele; determining whether said individual is unlikely to respond to selective serotonin reuptake inhibitor (SSRI) treatment by using information about the absence of homozygous major allele genotype at the polymorphism rs41271330 in the coding region of the BMP5 gene or absence of genotype in linkage disequilibrium with regards to major allele of rs41271330; and if said individual is unlikely to respond to selective serotonin reuptake inhibitor (SSRI) treatment due to the absence of homozygous major allele genotype at the polymorphism rs41271330 in the coding region of the BMP5 gene or a genotype in linkage disequilibrium with said allele, avoiding prescription to said individual a SSRI for the treatment of depressive disorders.

In an embodiment, escitalopram prescription is avoided, if absence of homozygous major allele genotype at the polymorphism rs41271330 in the coding region of the BMP5 gene or a genotype in linkage disequilibrium with said allele is identified in said individual.

To apply this method primers binding to the SEQ ID NO 1 or its complementary sequence SEQ ID NO: 2 can be used to amplify the genomic region containing rs41271330. Amplified region can be further genotyped by using variety of methods, for example Sanger sequencing, restriction fragment length polymorphism (RFLP), primer extension, but not limited to these.

SNP rs41271330 can be also genotyped by using predesigned Applied Biosystems Taqman SNP genotyping assay with ID C_3102261_10. In addition to these methods this SNP can be genotyped using the Sequenom iPlex assay with specific primers: primer1 (SEQ ID NO: 3) and primer2 (SEQ ID NO: 4). Also allele-specific PCR could be the method of choice. For this method two sets of primers are proposed: in the first set primer with SEQ ID NO: 5 is specific for A allele, primer with SEQ ID NO 6 is specific for G allele and primer with SEQ ID NO: 7 is a shared primer for both alleles; in the second set primer with SEQ ID NO: 9 is specific for A allele, primer with SEQ ID NO: 10 is specific for G allele and primer with SEQ ID NO: 8 is a shared primer for both alleles.

To implicate the present invention into practice, a kit for identification of an individual likely to respond to antidepressant treatment is provided, wherein said kit comprises a genotyping assay for detecting the genetic polymorphism rs41271330 in the coding region of the BMP5 gene or a genotyping assay for detecting a polymorphism that is in linkage disequilibrium with polymorphic alleles of rs41271330.

More specifically, a kit for identification of an individual likely to respond to selective serotonin reuptake inhibitor (SSRI) treatment is provided, wherein said kit comprises a genotyping assay for detecting the genetic polymorphism rs41271330 in the coding region of the BMP5 gene or a genotyping assay for detecting a polymorphism that is in linkage disequilibrium with polymorphic alleles at rs41271330.

The genotypic assay in this connection may contain, but is not limited to, appropriate primers, chemicals, nucleotides, instructions to isolate and detect nucleic acid sequences, hybridizing probes, sequencing of the respective nucleic acid region, or any other method known in the art capable of determining a nucleic acid residue at a particular position.

Most preferably, a kit for identification of an individual likely to respond to escitalopram treatment is provided, wherein said kit comprises a genotyping assay for detecting the genetic polymorphism rs41271330 in the coding region of the BMP5 gene or a genotyping assay for detecting a polymorphism that is in linkage disequilibrium with polymorphic alleles at rs41271330.

A person skilled in the art understands, that a polymorphism in a protein-coding sequence of a gene, which does not alter the amino acid in the encoded protein, may still cause alterations in the biological functions encoded by the particular region of the DNA. These alterations may be caused by (but are not limited to) modification of a binding site of a transcription factor; influencing the secondary structure of mRNA; influencing splicing of mRNA; any other mechanism leading to changes in the level of mRNA synthesis, lifespan or translation; as well as by a snRNA or miRNA encoded by the named region of DNA. Thus, another object of the invention is a method for modulating the expression of a biological molecule encoded by the BMP5 gene or by a sequence or an antisense sequence containing alleles in linkage disequilibrium with alleles at rs41271330 for changing the potential of an individual to respond to treatment with an antidepressant.

More particularly, the object of the invention is a method for modulating the expression of a biological molecule encoded by the BMP5 gene or by a sequence or an antisense sequence containing alleles in linkage disequilibrium with alleles at rs41271330 for changing the potential of an individual to respond to treatment with a SSRI.

Accordingly, the biological molecule responsible for the effect in a human's drug response to SSRI-s is a target for drug development to enhance said person's response to SSRI-s. Thus, another object of the invention is a molecule, which modulates the expression of a biological molecule encoded by the BMP5 gene or by a sequence or an antisense sequence containing alleles in linkage disequilibrium with alleles at rs41271330 leading to change in said person's response to antidepressants.

Another more particular object of the invention is a molecule, which modulates the expression of a biological molecule encoded by the BMP5 gene or by a sequence or an antisense sequence containing alleles in linkage disequilibrium with alleles at rs41271330 leading to change in said person's response to SSRI-s.

In a preferred embodiment, the object of the invention is a molecule, which modulates the expression of a biological molecule encoded by the BMP5 gene or by a sequence or an antisense sequence containing alleles in linkage disequilibrium with alleles at rs41271330 leading to change in said person's response to escitalopram.

### EXAMPLES

### EXAMPLE 1. Drug response phenotype studies

### Study subjects

The study sample consisted of 135 outpatients with Major Depressive Disorder, MDD (mean age 31.1 ± 11.6 years, 68% females) recruited at the Psychiatry Clinic of Tartu University Hospital in Estonia. The diagnosis according to DSM-IV criteria was verified using Mini International Neuropsychiatric Interview (M.I.N.I. 5.0.0) and substantiated by psychiatric history and medical records. At least moderate severity of depression was required for inclusion as indicated by a Montgomery-Asberg Depression Rating Scale (MADRS) total score of 22 or higher. MDD patients with a secondary current comorbid anxiety disorder were included in this study, except for obsessive-compulsive disorder, posttraumatic stress disorder, or panic disorder, and these comprised 51 % of the sample.

Patients were excluded if they met diagnostic criteria for any of the following: bipolar disorder, psychotic disorder or features, current eating disorders, mental retardation, any pervasive developmental disorder or cognitive disorder, or alcohol or drug abuse-related disorders within 12 months prior to baseline. Additional exclusion criteria were acute infections, neurological or any other unstable general disorders, serious suicide risk, formal behaviour therapy, or systematic psychotherapy, pregnancy or breastfeeding. None of patients was known to have a history of hypersensitivity or non-response to escitalopram or other SSRIs. The Human Studies Ethics Committee of the University of Tartu and State Agency of Medicines had approved the study protocol, which was in accordance with Declaration of Helsinki. All participants provided their written informed consent.

### Treatment and clinical assessment

The patients were treated with escitalopram 10-20 mg/day for 12 weeks using an open-label, placebo non-controlled study design. No other medications, including antiinflammatory drugs, were allowed during the study, except for hormonal contraceptives and zolpidem or zopiclone for insomnia. Clinical severity and treatment response were assessed bi-weekly using the MADRS and Clinical Global Impression scale (CGI). Hamilton Depression Scale (HAM-D) was also used as a secondary scale for clinical assessments. Additionally, the patients were asked to evaluate the depressive symptoms on the Beck Depression Inventory (BDI) and possible side effects on the Toronto Side-effect Scale (TSES) at each visit. All patients started treatment with escitalopram at a dose of 10 mg/day for the first 4 weeks. The patients showing at least 50% decline in the MADRS total score at week 4 continued taking 10 mg of escitalopram until the end of study. The dose of escitalopram was increased and kept at 20 mg in patients who demonstrated less than 50% decrease in MADRS total score at week 4 or who showed exacerbation of depressive symptoms in any of following visits. At the end of week 12 the patients were defined as responders if the decrease in both MADRS and HAM-D total scores was at least 50% and score on the CGI improvement scale was 2 or less. The remitters were defined if the scores were less than 12 on the MADRS and less than 8 on the HAM-D accordingly to earlier clinical trials. Patients who did not meet these criteria were defined as non-responders and non-remitters respectively. The severity of depressive symptoms and treatment response were rated blindly to genotypes by one psychiatrist. Each visit the patients were also asked to report about their regularity of taking the medication.

### Results

At the end of week 12 of treatment with escitalopram, 82 patients (60.7%) were defined as responders and 79 of them (in total 58.5%) achieved remission according to mentioned criteria for response and remission. As forty four patients (32.6%) showed insufficient or partial response to treatment and 9 patients (6.7%) discontinued escitalopram treatment due to lack of efficacy or side effects. The daily dose of escitalopram was increased and kept at 20 mg altogether in 85 patients from who 41 were classified as responders at the end of the study. As almost all responders fulfilled the criteria of remission, the analyses were carried out only between the groups of responders and non-responders. There were no significant differences in age or sex distribution between two groups, however various demographic and clinical assessments significantly differentiated responders from non-responders. Particularly, the non-responders had an earlier age of disease onset (p=0.01), were more melancholic (p=0.007), less drug-naïve (p=0.05) and had more comorbidity with anxiety disorders (p=0.04) than responders. At baseline, the severity of depression on the both MADRS (p=0.003) and BDI (p<0.001) was significantly higher among non-responders as compared with responders, although the number of previous depressive episodes and duration of current depressive episode were similar between the groups.

### EXAMPLE 2. Exome sequencing of individuals selected based on their drug response phenotype

### Selection of individuals for full exome sequencing

The selection of individuals for full exome sequencing was based on the drug response phenotype. 5 patients representing responders were all chosen from those showing good treatment response to lower dose (10 mg) of escitalopram daily whereas non-responders were selected from those who were resistant to treatment even after raising the dose to 20 mg daily at week 4 of the treatment. Additionally, all sequenced individuals were chosen to be women to eliminate the possible differences associated with sex. Taking into account the possibility that melancholic versus atypical subtype of depression may have impact on the association of some genetic factors with treatment outcome (1) we selected all 10 individuals among those who had melancholic features present.

### Measuring escitalopram concentration in blood

Escitalopram was determined by high performance liquid chromatography - triple quadrupole mass spectrometry. Samples (190 µl) were spiked with 10 µl d3-methadone internal standard (2 µg/ml in methanol) and extracted with 500 µl -20°C cold acetonitrile. Two µl supernatant were directly injected into the analysis instrumentation. Chromatographic separation of the analytes was performed on a reverse-phase C18 column (Waters Acquity® BEH C18, 2.1x50mm, Waters Corp. MA, USA) with a linear gradient of 50% acetonitrile / 50% 5mM formic acid in water to 100 % acetontrile over 3 min at a flow rate of 0.25 ml/min. Escitalopram was quantified by by triple quadrupole mass spectrometry on a Micromass Quattro® Ultima (Waters Corp. MA USA) using the mass transitions 325 > 109 m/z for escitalopram, and 313 > 268 m/z for the internal standard d3-methadone. The level of quantification for escitalopram was (LOQ) was set at 0.5 ng/ml (signal-to-noise ratio > 5).

### Targeted capture and exome sequencing

Genomic DNA was extracted from whole blood using the standard salt precipitation method. The DNA samples of five clear responders and five clear non-responders were subjected to exome capture using the NimbleGen 2.1M Exon Capture Array and Illumina sequencing as previously described. Each sample was sequenced to a mean depth of at least 22x, with sufficient depth to accurately call variants at ~95% of each targeted exome.

### Alignment of reads and detection of variants

Sequence reads of each individual were aligned to the human reference genome UCSC hg18 version (NCBI build 36.3) using SOAPaligner (15,16). Reads that had duplicated start sites were removed, the remaining reads mapped on or near target were collected for subsequent analysis and variant calling. SOAPsnp version 1.03 were used for assembling the consensus genotypes and identifying SNPs in target region (14). The parameters were set as "-i -d -o -r 0.0005 -e 0.001 -M -t -u -L -s - 2 -T ". A consensus genotype with Phred-like quality of at least 20 and at least four coverage depth was considered as the high-confident genotype. The genotypes that were different from the reference were extracted as candidate SNPs, and the final SNP results were filtered as follows: Phred-like SNP quality >= 20, overall depth from 4-200, copy number estimate < 2, and distance between two adjacent SNPs no less than 5.

### SNP genotyping

For genotyping exome sequencing findings in the Estonian MDD sample set, we designed a multiplex iPLEX Gold panel (Sequenom) for 10 of the SNPs that could be genotyped simultaneously. Pre-designed TaqMan assays (Applied Biosystems) were available for 21 of the remaining SNPs, while 6 SNPs and 1 novel polymorphism had to be genotyped by Sanger sequencing.

### Validation of findings

Replication sample consisted of 394 patients treated with escitalopram for 12 weeks. All the patients were of European origin. The assessment of treatment response was comparable with this used for original sample. For validation of significant markers from previous step 5 SNPs were genotyped with pre-designed Taqman assays and one SNP was genotyped with Sanger sequencing.

### Statistical analysis

All samples sequenced were also genotyped using the Illumina 370CNV array. We set a cut-off value for the quality score of genotypes so that concordance of genotypes between array and sequencing was 99.7%. In total, the two methods produced identical genotypes for 98% of the overlapping markers (n=10 430) on average. For exome genotypes with quality score¹⁶ of at least 20, the probability of being identical with the genotypes obtained using the 370CNV beadchip was 99.7%, whereas for the markers with quality score less than 20, the concordance was only 75%. Therefore we set a cut-off value for the quality score of genotypes used in next steps to be at least 20.

The markers were first selected from the exome sequencing data according to the following criteria: a) the genotypes of all 5 non-responders differed from those of the responders (complete separation); b) minor or major allele homozygotes were only present in either the responders or the non-responders; c) the minor allele was only present among the non-responders. As a result, 64 potential markers were selected for further study. Next, 38 markers were selected for replication, with the exclusion of excessive SNPs in complete linkage disequilibrium with each other, or SNPs with minor allele frequencies in non-responders that did not differ significantly (p>0.05) from responders.

These 38 markers were genotyped in the remaining 116 individuals, and tested for association with the clinical outcome. Three different outcome definitions were used: MADRS score at the final visit (week 12), relative change (percentage) in MADRS score since baseline, and absolute change in MADRS score since baseline. For each outcome, a univariate additive regression model was fitted to assess the association, as well as a multivariate model, adjusted for age, gender, age at first depression episode, baseline MADRS and BDI scores. Additionally a mixed-effects longitudinal model was fit for MADRS scores at week 0 to week 12, using a random subject-specific intercept. Also here, both unadjusted and adjusted models were fit. Age at recruitment, gender, age at first depression episode and baseline BDI score appeared to be independently predictive (p<0.05) for at least one of the outcomes after adjusting for other covariates in a multivariate regression model and were therefore used as adjustment variables for genotype-outcome association. As a result, 5 markers were selected for further study that showed the same direction of association as in the discovery sample and had a significant (p<0.01) association with the outcome in at least one of the analyses.

The 5 selected markers were further tested in the replication dataset, using only relative change in MADRS scores as the univariate outcome and the 7 consecutive MADRS scores as the longitudinal outcome. In the adjusted analyses, the same adjustment variables were used as in the Estonian replication set.

### Results

From a sample of 126 MDD patients treated with SSRI escitalopram for 12 weeks, we selected five of both well-characterized responders and non-responders for full exome sequencing. The responders were patients who demonstrated consistent and significant decrease in depression symptoms with a daily dose of 10 mg of escitalopram during 12 weeks, while the non-responders were resistant to treatment even after raising the dose to 20 mg/day from week 4 of treatment. We confirmed that the patients had adhered to the treatment protocol by measuring the levels of escitalopram in blood samples taken before treatment and then at week 4 and 12, respectively. We enriched exonic sequences using the NimbleGen 2.1M Exon Capture Array and performed Illumina paired-end sequencing. Each sample was sequenced to a mean depth of at least 22x, with sufficient depth to accurately call variants at ~95% of each targeted exome. All samples were also genotyped using the Illumina 370CNV array. We set a cut-off value for the quality score of genotypes so that concordance of genotypes between array and sequencing was 99.7% and therefore the quality score of genotypes used in next steps was at least 20. Using the filtered data, we searched for markers that could distinguish responders from non-responders according to the following criteria: a) all 5 non-responders genotypes different from the responders genotypes (complete separation); b) minor allele homozygotes only present in either responders or non-responders; c) minor allele only present in non-responders (as non-response is a less frequent event than response, one may expect minor alleles of any potential effect markers to increase the probability of non-response). Altogether, these criteria were fulfilled for 64 markers, all of which were SNPs and only 2 of these were not present in the latest build (135) of dbSNP. We found no insertions or deletions that could distinguish responders from non-responders based on these three criteria.

In the next step, 38 markers were selected for further genotyping in samples from the remaining 116 Estonian MDD patients, with the exclusion of redundant SNPs in complete linkage disequilibrium with each other and SNPs where the differences in minor allele counts between responders and non-responders were not statistically significant at p < 0.05 (Fisher's exact test for allele frequencies).

To assess the effect of each of the 38 selected markers on the clinical outcome in the set of 116 Estonian MDD patients, unadjusted and adjusted regression models were fit for the relative change in the MADRS score since baseline. In addition, longitudinal analyses were conducted to estimate the effect on the outcome (MADRS score) profile from baseline to week 12. For adjustment, age, gender, age at first depression episode, baseline MADRS and Beck Depression Inventory (BDI) scores were used as covariates. Altogether, 5 markers (Tables 1) showed the same direction of association as in the exome sequencing sample set, and had a significant (p<0.01) association with the outcome in either cross-sectional or longitudinal unadjusted analysis (Table 1). Next, the 5 SNPs which could distinguish the responders from the non-responders in the 116 Estonian MDD patients were further genotyped in replication sample set, comprising of 394 MDD patients treated with escitalopram. During this validation step rs41271330 in the bone morphogenetic protein 5 (BMP5) gene was significantly associated with escitalopram drug response (p<0.001 for both Estonian and replication samples), with the effect allele being the same as in the Estonian sample set (Table 1): relative MADRS score reduction from baseline decreased in average by 18% (SE=6%) per each copy of the minor allele in the Estonian sample and by 7% (SE=3%) in the replication sample.

### EXAMPLE 3 Patients with major depression carrying the rs41271330 homozygous minor allele (A/A) or heterozygous minor allele (A/G) genotype are less likely to benefit from escitalopram treatment

As the association of rs41271330 with treatment outcome replicated, we analyzed the effect of this SNP on escitalopram efficacy more closely in the Estonian replication sample. The 52% (14/27) of patients carrying the minor allele (A) of rs41271330 had moderate or severe depression (MADRS score 16 or more) by the end of the 12-week treatment period, compared to 22% (19/89) patients homozygous for the major allele (G) (Table 2). Also, patients with the G/G genotypes could be clearly distinguished as having the lowest MADRS scores across all 6 follow-up visits (Figure 1a) and the greatest relative decrease in MADRS scores from baseline (Figure 1b). The A/A and A/G genotypes of rs41271330 were also predictors of the need for higher escitalopram dose after the initial 4 weeks of treatment - 62.9% of the patients homozygous for the major allele needed an increased dose compared to 88.9% of the patients carrying the minor allele (p=0.010). Therefore, rs41271330 and markers in linkage disequilibrium with this SNP have potential to be used as predictors of SSRI response and the protein or RNA molecule encoded by the BMP5 gene may act as an important factor in antidepressant action. This finding can be considered as clinically relevant because the frequency of the minor allele (A) of the rs41271330 polymorphism is approximately 15% in the Caucasian population, which means that almost 28% of the total population have A/A or A/G genotypes and are therefore more likely to get unsatisfactory results from SSRI treatment.

**Table 1. SNPs associated with escitalopram treatment outcome in Estonian MDD sample set and their effect on treatment response in replication sample set.**

| | Unadjusted analysis | | | | | | | |
|---|---|---|---|---|---|---|---|---|
| | Estonian sample | | | | Replication sample | | | |
| | Cross-sectional | | Longitudinal | | Cross-sectional | | Longitudinal | |
| | Beta (SE) | p-value | Beta (SE) | p-value | Beta (SE) | p-value | Beta (SE) | p-value |
| rs 17599 | -0.09 | | 0.49 | | 0.02 | | 0.02 | |
| | (0.05) | 0.10 | (0.17) | 0.005 | (0.03) | 0.41 | (0.03) | 0.534 |
| rs35864197 | 0.12 | | -0.69 | | 0.005 | | 0.12 | |
| | (0.06) | 0.04 | (0.19) | 0.0002 | (0.03) | 0.85 | (0.04) | 0.0007 |
| rs2302566 | 0.12 | | -0.89 | | -0.04 | | 0.05 | |
| | (0.10) | 0.25 | (0.33) | 0.006 | (0.04) | 0.30 | (0.06) | 0.403 |
| rs41271330 | -0.17 | | 0.66 | | -0.07 | | 0.13 | |
| | (0.06) | 0.006 | (0.20) | 0.001 | (0.03) | 0.03 | (0.04) | 0.001 |
| rs79901896 | -0.17 | | 0.85 | | 0.05 | | -0.25 | |
| | (0.09) | 0.06 | (0.30) | 0.004 | (0.04) | 0.23 | (0.06) | 0.00001 |

| | Adjusted analysis | | | | | | | |
|---|---|---|---|---|---|---|---|---|
| | Estonian sample | | | | Replication sample | | | |
| | Cross-sectional | | Longitudinal | | Cross-sectional | | Longitudinal | |
| | Beta (SE) | p-value | Beta (SE) | p-value | Beta (SE) | p-value | Beta (SE) | p-value |
| rs17599 | -0.05 | | 0.49 | | 0.03 | | 0.02 | |
| | (0.05) | 0.389 | (0.18) | 0.006 | (0.03) | 0.324 | (0.03) | 0.565 |
| rs35864197 | 0.12 | | -0.77 | | -0.01 | | 0.12 | |
| | (0.06) | 0.036 | (0.20) | 0.0001 | (0.03) | 0.704 | (0.04) | 0.0009 |
| rs2302566 | 0.03 | | -0.89 | | -0.04 | | 0.04 | |
| | (0.10) | 0.759 | (0.33) | 0.007 | (0.04) | 0.389 | (0.06) | 0.463 |
| rs41271330 | -0.12 | | 0.65 | | -0.05 | | 0.13 | |
| | (0.06) | 0.051 | (0.20) | 0.001 | (0.03) | 0.085 | (0.04) | 0.001 |
| rs79901896 | -0.21 | | 0.84 | | 0.04 | | -0.25 | |
| | (0.08) | 0.016 | (0.30) | 0.005 | (0.04) | 0.322 | (0.06) | 0.00001 |

**Table 2. Distribution of rs41271330 genotypes between different MADRS score groups after 12 weeks of treatment**

| rs41271330 genotype | MADRS score at week 12 | | | |
|---|---|---|---|---|
| | 0...7 | 8...15 | 16...25 | >25 |
| G/G | 61 | 9 | 15 | 4 |
| | 69% | 10% | 17% | 4% |
| A/G + A/A | 9 | 4 | 10 | 4 |
| | 33% | 15% | 37% | 15% |

MADRS (Montgomery-Asberg Depression Rating Scale) - diagnostic questionnaire to measure the severity of depressive episodes. Score "0" indicates that no symptoms of depression were present.

Changes in the average MADRS score values during the escitalopram treatment across rs41271330 genotype groups in the Estonian MDD sample set is shown in Figure 1A. Relative changes in the average MADRS score values during the escitalopram treatment across rs41271330 genotype groups in the Estonian MDD sampleset is shown in Figure 1A

### References

1. Baune, B.T. et al. Serotonin receptor 1A-1019C/G variant: impact on antidepressant pharmacoresponse in melancholic depression? Neurosci Lett 436, 111-5 (2008).
2. Beck, H.N., Drahushuk, K., Jacoby, D.B., Higgins, D. & Lein, P.J. Bone morphogenetic protein-5 (BMP-5) promotes dendritic growth in cultured sympathetic neurons. BMC Neurosci 2, 12 (2001).
3. Candilis, P.J. et al. Quality of life in patients with panic disorder. J Nerv Ment Dis 187, 429-34 (1999).
4. Eaton, W.W., Kessler, R.C., Wittchen, H.U. & Magee, W.J. Panic and panic disorder in the United States. Am J Psychiatry 151, 413-20 (1994).
5. Faravelli, C., Paterniti, S. & Scarpato, A. 5-year prospective, naturalistic follow-up study of panic disorder. Compr Psychiatry 36, 271-7 (1995).
6. Furuta, Y., Piston, D.W. & Hogan, B.L. Bone morphogenetic proteins (BMPs) as regulators of dorsal forebrain development. Development 124, 2203-12 (1997).
7. Garriock, H.A. et al. A genomewide association study of citalopram response in major depressive disorder. Biol Psychiatry 67, 133-8 (2010).
8. Ising, M. et al. A genomewide association study points to multiple loci that predict antidepressant drug treatment outcome in depression. Arch Gen Psychiatry 66, 966-75 (2009).
9. Jamison, K.R. Suicide and bipolar disorder. J Clin Psychiatry 61 Suppl 9, 47-51 (2000).
10. Katerndahl, D.A. & Realini, J.P. Quality of life and panic-related work disability in subjects with infrequent panic and panic disorder. J Clin Psychiatry 58, 153-8 (1997).
11. Kato, M. & Serretti, A. Review and meta-analysis of antidepressant pharmacogenetic findings in major depressive disorder. Mol Psychiatry 15, 473-500 (2010).
12. Kessler, R.C. et al. Lifetime panic-depression comorbidity in the National Comorbidity Survey. Arch Gen Psychiatry 55, 801-8 (1998).
13. Lein, P.J. et al. Glia induce dendritic growth in cultured sympathetic neurons by modulating the balance between bone morphogenetic proteins (BMPs) and BMP antagonists. J Neurosci 22, 10377-87 (2002).
14. Li, R. et al. SNP detection for massively parallel whole-genome resequencing. Genome Res 19, 1124-32 (2009).
15. Li, R., Li, Y., Kristiansen, K. & Wang, J. SOAP: short oligonucleotide alignment program. Bioinformatics 24, 713-4 (2008).
16. Li, R. et al. SOAP2: an improved ultrafast tool for short read alignment. Bioinformatics 25, 1966-7 (2009).
17. Mehler, M.F., Mabie, P.C., Zhang, D. & Kessler, J.A. Bone morphogenetic proteins in the nervous system. Trends Neurosci 20, 309-17 (1997).
18. Noyes, R. & Perry, P. Maintenance treatment with antidepressants in panic disorder. J Clin Psychiatry 51 Suppl A, 24-30 (1990).
19. Nutt, D.J. Rationale for, barriers to, and appropriate medication for the long-term treatment of depression. J Clin Psychiatry 71 Suppl E1, e02 (2010).
20. Solloway, M.J. & Robertson, E.J. Early embryonic lethality in Bmp5;Bmp7 double mutant mice suggests functional redundancy within the 60A subgroup. Development 126, 1753-68 (1999).
21. Tilleman, H. et al. Bmp5/7 in concert with the mid-hindbrain organizer control development of noradrenergic locus coeruleus neurons. Mol Cell Neurosci 45, 1-11(2010).
22. Trivedi, M.H. et al. Medication augmentation after the failure of SSRIs for depression. N Engl J Med 354, 1243-52 (2006).
23. Uher, R. et al. Genome-wide pharmacogenetics of antidepressant response in the GENDEP project. Am J Psychiatry 167, 555-64 (2010).
24. Vogel-Höpker, A. & Rohrer, H. The specification of noradrenergic locus coeruleus (LC) neurones depends on bone morphogenetic proteins (BMPs). Development 129, 983-91 (2002).
25. Weissman, M.M. et al. Cross-national epidemiology of major depression and bipolar disorder. JAMA 276, 293-9 (1996).
26. Wittchen, H.U. & Essau, C.A. Epidemiology of panic disorder: progress and unresolved issues. J Psychiatr Res 27 Suppl 1, 47-68 (1993).

## Claims

1. A method to determine potential response to antidepressant treatment comprising the steps of:
a) obtaining a biological sample from a human subject;
b) amplifying genomic region containing rs41271330 by using primers hybridizing with SEQ ID NO:1 or SEQ ID NO:2 or with a haplotype block specific to rs41271330;
c) determining a presence or an absence of a major or a minor allele of polymorphism rs41271330 in coding region of each allele of subject's BMP5 gene, or determining alleles in linkage disequilibrium with a major or a minor allele of rs41271330 at a polymorphic position in haplotype block containing rs41271330; and
d) assessing homozygosity of the major allele of the polymorphism rs41271330 in the coding region of the BMP5 gene, wherein homozygosity of the major allele of the polymorphism rs41271330 in the coding region of the BMP5 gene or a genotype in linkage disequilibrium with the homozygous major allele genotype indicates potential response.

2. The method of Claim 1, wherein the potential response is to selective serotonin reuptake inhibitor (SSRI) treatment.

3. A method for managing therapy of a human subject comprising the steps of:
a) obtaining a biological sample from the human subject in need of antidepressant treatment;
b) amplifying genomic region containing rs41271330 by using primers hybridizing with SEQ ID NO:1 or SEQ ID NO:2 or with a haplotype block specific to rs41271330;
c) determining a presence or an absence of major or minor allele of polymorphism rs41271330 in coding region of each allele of the subject's BMP5 gene, or determining alleles in linkage disequilibrium with major or minor allele of rs41271330 at a polymorphic position in the haplotype block containing rs41271330;
d) assessing homozygosity of major allele of the polymorphism rs41271330 in the coding region of the BMP5 gene, wherein the presence of homozygous major allele of polymorphism rs41271330 in the coding region of the BMP5 gene or a genotype in linkage disequilibrium with the homozygous major allele genotype indicates potential response to selective serotonin reuptake inhibitor (SSRI) treatment; and
e) prescribing SSRIs to the subject having homozygous major allele genotype of polymorphism rs41271330 or a genotype in linkage disequilibrium with said allele.

4. The method of Claim 1 or 3, wherein the potential response is response to escitalopram treatment.

5. A method for managing therapy of a human subject comprising the steps of:
a) obtaining a biological sample from the human subject in need of antidepressant treatment;
b) amplifying genomic region containing rs41271330 by using primers hybridizing with SEQ ID NO:1 or SEQ ID NO:2 or with a haplotype block specific to rs41271330;
c) determining a presence or an absence of a major or a minor allele of the polymorphism rs41271330 in the coding region of each allele of the subject's BMP5 gene, or determining alleles in linkage disequilibrium with the major or the minor allele of rs41271330 at a polymorphic position in the haplotype block containing rs41271330;
d) assessing homozygosity of the major allele of the polymorphism rs41271330 in the coding region of the BMP5 gene, wherein absence of homozygous major allele genotype of the polymorphism rs41271330 in the coding region of the BMP5 gene or absence of a genotype in linkage disequilibrium with said major allele indicates potential nonresponse to selective serotonin reuptake inhibitor (SSRI) treatment; and
e) avoiding prescribing SSRI treatment to the subject not having homozygous major allele genotype of polymorphism rs41271330 in the coding region of the BMP5 gene nor a genotype in linkage disequilibrium with said allele.

6. The method of Claim 5, wherein treatment with escitalopram is avoided.

7. A kit for identification of individuals likely to respond to antidepressant treatment, wherein said kit comprises a genotyping assay for detecting the genetic polymorphism rs41271330 in the coding region of the BMP5 gene or a genotyping assay for detecting a polymorphism that is in linkage disequilibrium with polymorphic alleles of the polymorphism rs41271330.

8. The kit of claim 7, wherein the kit comprises:
a) primers for amplifying genomic region containing rs4171330;
b) chemicals and nucleotides to isolate and detect nucleic acid sequences;
c) hybridizing probes; and
d) instructions to determine nucleic acid residue at particular position.

9. The kit of claim 7, wherein the kit is for identification of individuals likely to respond to selective serotonin reuptake inhibitor (SSRI) treatment.

10. The kit of claim 7, wherein the kit is for identification of individuals likely to respond to escitalopram treatment.

11. A method for modulating a potential of an individual to respond to the treatment with an antidepressant, said method comprising a step of modulating expression of a biological molecule encoded by the BMP5 gene or by a sequence or an antisense sequence containing alleles in linkage disequilibrium with alleles of the polymorphism rs41271330.

12. The method of Claim 11, wherein potential to respond to selective serotonin reuptake inhibitor (SSRI) treatment is being modulated.

13. The method of Claim 11, wherein the potential to respond to escitalopram treatment is being modulated.

14. A molecule, which modulates the expression of a biological molecule encoded by the BMP5 gene or by a sequence or an antisense sequence containing alleles in linkage disequilibrium with alleles of rs41271330.

15. The molecule of claim 14, wherein the molecule is used to modulate the response of a human subject to the compounds selected from the group consisting of antidepressants, serotonin reuptake inhibitors (SSRIs) and escitalopram.
